(19) Europäisches Patentamt
European Patent Office
Office européen des brevets

(11) EP 0 805 135 B1

(12) **FASCICULE DE BREVET EUROPEEN**

(45) Date de publication et mention de la délivrance du brevet:
**29.11.2000 Bulletin 2000/48**

(51) Int Cl.[7]: **C07C 17/087**, C07C 19/08

(21) Numéro de dépôt: **97201260.3**

(22) Date de dépôt: **25.04.1997**

(54) **Procédé pour la préparation du 2-h-heptafluoropropane**

Verfahren zur Herstellung von 2-H-Heptafluorpropan

Process for the preparation of 2-H-heptafluoropropane

(84) Etats contractants désignés:
**DE ES FR GB GR IT NL**

(30) Priorité: **29.04.1996 DE 19617091**

(43) Date de publication de la demande:
**05.11.1997 Bulletin 1997/45**

(73) Titulaire: **SOLVAY (Société Anonyme)**
**1050 Bruxelles (BE)**

(72) Inventeurs:
- **Vollmueller, Helmut**
  **55130 Mainz (DE)**
- **Franz, Raimund**
  **65779 Kelkheim (DE)**
- **Siegemund, Günther**
  **65719 Hofheim (DE)**

(74) Mandataire: **Jacques, Philippe et al**
**Solvay S.A.**
**Département Propriété Industrielle**
**310, rue de Ransbeek**
**1120 Bruxelles (BE)**

(56) Documents cités:
**EP-A- 0 634 383**
**EP-A- 0 634 384**
**WO-A-97/11042**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

L'invention concerne un procédé pour la préparation du 2-H-heptafluoropropane à partir d'hexafluoropropène dans une colonne à bulles.

Le 2-H-heptafluoropropane (R 227) est un agent propulseur et un réfrigérant précieux à l'échelle technique, exempt de chlore et par conséquent inoffensif pour la couche d'ozone stratosphérique. On le prépare de manière avantageuse en se référant à la formule développée (I) par addition de fluorure d'hydrogène à de l'hexafluoropropène.

$$CF_3\text{-}CF = CF_2 + HF \rightarrow CF_3\text{-}CHF\text{-}CF_3 \qquad (I)$$

A titre d'exemple, on connaît, d'après le document GB-902 590, le fait de mettre cette réaction en oeuvre à une température de 250 à 450°C sur un catalyseur de charbon actif. Contrairement au procédé de ce type catalysé en lit fixe, on propose, dans le document EP-A-0 634 383, d'introduire l'hexafluoropropène gazeux dans un réacteur équipé de colonnes à bulles uniquement à une température de 20 à 80°C, le réacteur contenant, comme milieu réactionnel et comme catalyseur, un hydrofluorure complexe liquide d'une base azotée organique répondant à la formule générale

$$[B \bullet nHF] \qquad (II)$$

où n représente un nombre entier ou une fraction ≤ 4 et B représente une base azotée organique.

De cette manière, une molécule de HF provenant de l'hydrofluorure s'ajoute à une molécule d'hexafluoropropène, si bien qu'à la surface du liquide, le 2-H-heptafluoropropane gazeux s'échappe sous forme de produit réactionnel. Le milieu réactionnel est guidé en circuit fermé via un conduit tubulaire externe et est régénéré dans ces circonstances par addition de fluorure d'hydrogène en continu.

De manière habituelle, on obtient la dispersion fine d'un gaz guidé dans une colonne à bulles, requise pour une mise en réaction rapide et complète, par exemple au moyen d'une fritte constituée par une matière frittée ou encore via un agitateur. Toutefois, lors de l'hydrofluoration d'hexafluoropropène en heptafluoropropane dans le complexe liquide (II), des difficultés interviennent, qui sont liées au degré de solubilité des deux gaz dans ce milieu réactionnel. Dans des conditions réactionnelles typiques, l'hexafluoropropène est soluble à concurrence de 44 g/(kg.bar) et l'heptafluoropropane même jusqu'à concurrence de 63 g/(kg.bar). Par conséquent, une dispersion intense peut avoir pour conséquence une dissolution complète des gaz et une disparition des bulles. Toutefois, ceci donne lieu à une sursaturation du milieu réactionnel avec l'heptafluoropropane qui ne s'échappe plus du liquide que de manière irrégulière et par à-coups, cette sursaturation pouvant amener une formation de mousse excessive. En outre, il peut arriver que du gaz se libère à des endroits indésirables, par exemple dans la pompe de circulation pour le milieu réactionnel. Dans ce cas, la capacité de refoulement de la pompe diminue. Il est vrai que l'on peut empêcher la sursaturation et ses conséquences en augmentant le débit du gaz, ce qui augmente le nombre de bulles de gaz ou bien en diminuant le degré de dispersion, si bien que l'on obtient des bulles plus grosses; toutefois, ces mesures amènent le plus souvent une diminution de la conversion, comme démontré ci-dessous dans un exemple de comparaison. Par conséquent, l'objectif consistant à maintenir la concentration d'hexafluoropropène n'ayant pas réagi dans les produits gazeux de réaction à une valeur inférieure à 100 ppm ne peut être atteint que dans les conditions déterminées et délimitées de manière très étroite.

Dans la demande de brevet allemand non publiée P 195 34 917.2-4 (WO 97/11042), on a proposé, lors de la mise en oeuvre de ce procédé, d'élever la pression dans le réacteur à une valeur de 1,3 à 10 bar à l'aide d'une soupape d'étranglement pour ainsi maintenir complètement les deux gaz en solution. Ainsi, on obtient une conversion complète de l'hexafluoropropène; en outre, l'heptafluoropropane se libère de manière fiable lors de la détente du milieu réactionnel en circulation derrière la soupape d'étranglement.

Toutefois, dans de telles conditions réactionnelles, l'hexafluoropropène est à même de réagir non seulement avec HF, mais aussi avec lui-même pour former des homologues di- et trimères de l'hexafluoropropène. Cette tendance à l'auto-addition dépend de la concentration et est par conséquent favorisée par une pression supérieure. Il est vrai que l'auto-addition est encore très minime jusqu'à une surpression de 0,7 bar (mesurée au bas du réacteur); toutefois, au-dessus de cette valeur, elle augmente de manière notable et, dans le procédé selon le document P 195 34917.2-4 (WO 97/11042), elle n'est acceptable que dans un intervalle de surpression étroit qui doit constamment être surveillé. De manière simultanée, on impose par là-même, au débit du gaz, une limite supérieure nette, étant donné qu'au-dessus de cette limite, sous pression constante, on obtiendrait à nouveau des bulles alors que, dans ce procédé, le milieu réactionnel doit rester exempt de bulles. Les hexafluoropropènes di- et trimères que l'on obtient à des pressions

supérieures ne réagissent pas par addition avec le HF provenant du milieu réactionnel. Etant donné qu'ils sont partiellement toxiques, l'heptafluoropropane doit en être séparé via une distillation fractionnée, soignée et coûteuse.

Par conséquent, l'objet était de trouver un procédé utilisant des colonnes à bulles pour la préparation d'heptafluoropropane à partir d'hexafluoropropène et de HF, dans lequel les problèmes sus-mentionnés n'interviennent pas, procédé dans lequel on obtient, également en l'absence d'une dissolution complète des gaz forcée via une soupape d'étranglement sous pression élevée, une conversion quantitative de l'hexafluoropropène et une libération fiable d'heptafluoropropane pur. Cet objet est réalisé conformément à l'invention en utilisant une colonne à bulles équipée de manière spécifique.

L'objet de l'invention concerne dès lors un procédé pour la préparation du 2-H-heptafluoropropane à partir d'hexafluoropropène et de fluorure d'hydrogène dans un appareil équipé de colonnes à bulles remplies avec, à titre de milieu réactionnel, un hydrofluorure liquide d'une base azotée organique répondant à la formule générale

$$[B \cdot nHF] \qquad (II)$$

où B représente une base azotée organique et n représente un nombre entier ou une fraction $\leq 4$ , de préférence > 2 et < 3, caractérisé en ce qu'on utilise une colonne à bulles munie d'un système de recyclage du milieu réactionnel et comprenant une partie de base (B), un tube de réaction (*T*) et une partie de tête (*H*), dans lequel le tube de réaction (T) est équipé d'au moins une résistance hydraulique provoquant une accélération de l'écoulement du milieu réactionnel liquide d'un facteur d'au moins 20 lors de son passage à travers celle-ci.

Généralement, on utilise une résistance hydraulique entraînant une accélération de l'écoulement du milieu liquide ne dépassant pas 200. Les résistances hydrauliques provoquant une accélération de l'écoulement de 50 à 100 sont préférées.

Comme résistance hydraulique, on peut mettre en oeuvre tout dispositif permettant d'accélérer le milieu réactionnel dans les proportions ci-dessus. On utilise avantageusement une plaque disposée dans le tube de réaction (T) de manière à occuper toute sa section et percée de petits orifices, ci-après dénommés alésages. Généralement, cette plaque est disposée dans le tube de réaction (T) transversalement et les alésages sont disposés selon l'axe du tube de réaction (T). Ils s'étendent donc de préférence en direction axiale par rapport au tube de réaction (T). De préférence, les alésages sont constitués d'orifices cylindriques, présentant une longueur au moins égale et au maximum le double de leur diamètre. Un rapport de la longueur au diamètre d'environ 2 est préféré. A titre d'exemple, on peut utiliser des alésages présentant un diamètre de 0,1 à 1 cm. On calcule le nombre des alésages nécessaires de telle sorte que l'écoulement du milieu réactionnel liquide (sans tenir compte des bulles de gaz) soit accéléré au moins d'un facteur 20 lors de son passage à travers les alésages. Pour ce faire, il faut que le rapport de l'aire de section totale des alésages d'une résistance hydraulique à l'aire de section du tube de réaction (T) s'élève au plus à 0,05.

De préférence, le tube de réaction (T) est équipé d'au moins deux résistances hydrauliques, l'une étant située près de la partie de base (B) et l'autre près de la partie de tête (H) de la colonne à bulles. Lorsque la longueur du tube de réaction (T) dépasse 2 m, on l'équipe de préférence en outre d'une ou de plusieurs résistances hydrauliques intermédiaires.

Aux fins de la présente invention, la vitesse d'écoulement du milieu réactionnel est exprimée sur base liquide, c'est-à-dire sans tenir compte du volume occupé par les bulles de gaz. Habituellement, on fait circuler le milieu réactionnel dans la colonne à bulles avec une vitesse d'écoulement dans le tube de réaction (T) ($V_T$) de 1 à 20 cm/s. De préférence, cette vitesse d'écoulement se situe de 2 à 10 cm/s. De manière particulièrement préférée, elle est de 3 à 7 cm/s. En revanche, la vitesse d'écoulement du milieu réactionnel lors de son passage à travers chaque résistance hydraulique ($V_C$) représente de 20 à 200 fois la valeur $V_T$. De préférence, $V_C$ s'élève au moins à 200 cm/s.

En maintenant une vitesse d'écoulement $V_T$ d'au moins 1 cm/s et une vitesse $V_C$ d'au moins 200 cm/s, le procédé selon l'invention présente trois caractéristiques:

1) à chaque résistance hydraulique, on obtient toujours un grand nombre de bulles de gaz très finement divisées,
2) la formation de bulles de gaz aux résistances hydrauliques est indépendante de la solubilité et du degré de dissolution des gaz dans le milieu réactionnel,
3) à la résistance hydraulique située la plus en haut, l'heptafluoropropane se libère complètement du liquide sous forme gazeuse.

La hauteur du niveau de liquide à maintenir dans la colonne à bulles mise en oeuvre dans le procédé selon l'invention est délimitée d'une part par le temps de séjour minimal requis du substrat dans le milieu réactionnel et d'autre part, par la pression hydrostatique s'exerçant sur le bas de la colonne. Elle se situe de préférence de 2,5 à 4 m, en particulier à 3 à 3,5 m. Le niveau du liquide réactionnel se situe en général à 20 - 80 cm, de préférence 20 - 40 cm, au-dessus de la résistance hydraulique située la plus en haut.

De manière avantageuse, on sélectionne un débit élevé d'hexafluoropropène de telle sorte qu'en plus du milieu réactionnel liquide, des bulles de gaz pénètrent également dans les résistances hydrauliques. Ainsi, on augmente fortement la capacité de l'appareil par rapport à celle décrite dans le document P 195 34 917.2-4 (WO-97/11042): le domaine du débit utilisable en pratique, avec un diamètre interne donné du réacteur, n'est plus limité que par le temps de séjour minimal requis du substrat dans le milieu réactionnel, c'est-à-dire par la hauteur du réacteur. La surpression de liquide maximale qui intervient dans la partie de base *B* du réacteur peut être maintenue à une valeur inférieure à 0,7 bar en réglant une capacité appropriée de la pompe de circulation et est par conséquent toujours inférieure à celle régnant dans l'appareil selon le document P 195 34 917.2-4 (WO 97/11042). La formation d'homologues d'hexafluoropropène di- et trimères reste très minime.

Le débit maximal possible de l'hexafluoropropène dépend de l'aire de section du tube de réaction (T) de la colonne à bulles. Il peut s'élever à 100 g par heure et par $cm^2$ de section du tube de réaction (T). En pratique, on préfère un débit de 40 à 80 g/($cm^2$.h).

Dans un mode de fonctionnement préféré du procédé selon l'invention, on utilise une colonne à bulles munie d'un tube de réaction (*T*) possédant une longueur de 200 à 350 cm, de préférence de 250 à 300 cm, et un diamètre interne de 3 à 50 cm, de préférence de 5 à 20 cm, équipé, à son extrémité inférieure, à mi-hauteur et à son extrémité supérieure de trois résistances hydrauliques. De préférence, la résistance hydraulique médiane se trouve, lorsqu'on répartit la distance entre les résistances hydrauliques extrêmes en 100 parties égales, entre le tronçon de distance 35 et le tronçon de distance 65.

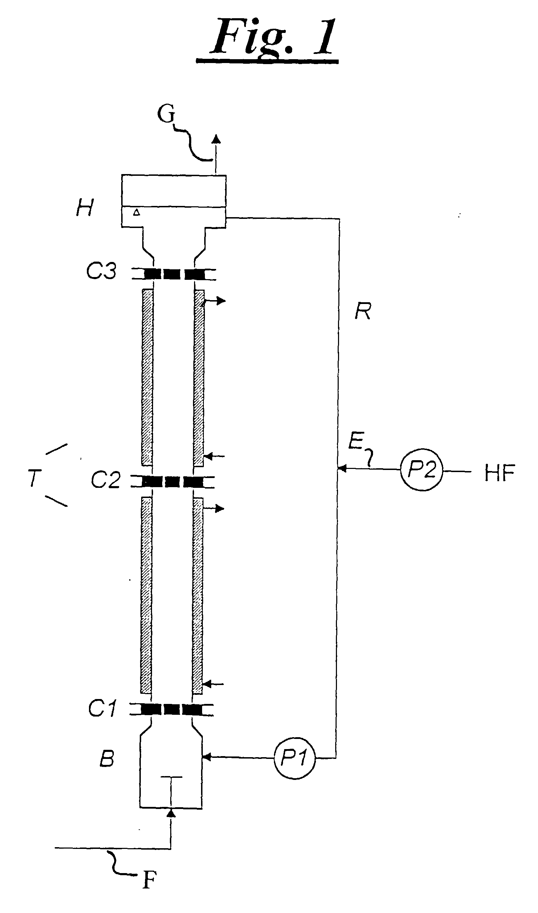

Un appareil approprié à ce mode de fonctionnement préféré est décrit ci-après et est représenté de manière schématique en figure 1.

La colonne à bulles est constituée par une partie de base *B*, par un tube de réaction *T* qui est constitué par des parties qui peuvent être chauffées de l'extérieur avec de l'eau chaude, et par une partie de tête *H*. A l'extrémité inférieure, à mi-hauteur et à l'extrémité supérieure du tube de réaction *T*, on applique au total trois résistances hydrauliques *C1*, *C2* et *C3* qui présentent chacune des alésages. Le niveau du liquide réactionnel est désigné par Δ. Le conduit tubulaire *R*, ainsi que la pompe *P1* servent au recyclage du contenu liquide du réacteur pour faire en sorte qu'il traverse les alésages des résistances hydrauliques de manière constamment ascendante. Le conduit d'alimentation *E* sert à l'introduction en continu de manière dosée de fluorure d'hydrogène liquide dans le tube *R* au moyen de la pompe *P2*. Pour le conduit d'alimentation *E* et pour son environnement, on a besoin d'une matière résistant à HF. Pour toutes les autres parties de l'appareil, on peut utiliser, outre de l'acier ou des matières synthétiques en polyoléfines, également du verre de borosilicate. Le conduit d'alimentation F sert à l'introduction en continu de manière dosée d'hexafluoropropène, de préférence à l'état gazeux. Pour le contrôle de l'alimentation de l'hexafluoropropène et de HF, on prévoit des dispositifs de pesée et de mesure du débit correspondants. Le conduit G sert à soutirer l'heptafluoropropane gazeux.

Comme on l'a déjà mentionné dans l'introduction, l'hydrofluoration de l'hexafluoropropène se déroule conformément au document EP-A-0 634 383 dans le domaine de température réactionnelle de 20 à 80°C. Sur base de la viscosité du milieu réactionnel, on préfère un domaine de 60 à 75°C.

Des hydrofluorures appropriés, liquides à ces températures, répondant à la Formule (II), sont par exemple le [$(CH_3)_3N$ x n HF], le [$(C_2H_5)_3N$ x n HF], le [$(C_4H_9)_3N$ x n HF], de même que

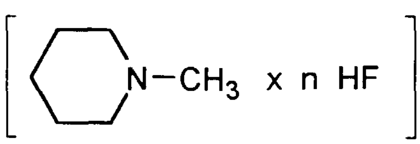

,

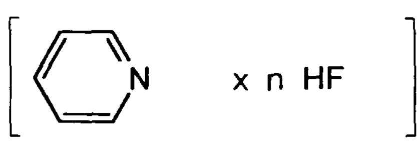

et

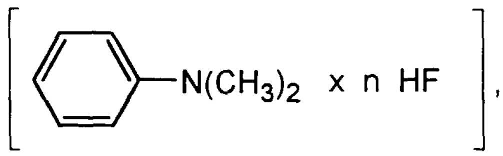

avec 2 < n < 3.

De préférence on utilise un des trois hydrofluorures mentionnés en premier lieu. Mais également tous les autres hydrofluorures répondant à la Formule (II), mentionnés dans le document EP-A-0 634 383, qui sont liquides aux températures de réaction choisies, sont appropriés. La préparation des hydrofluorures est également décrite dans le document EP-A-0 634 383, auquel on fait ici référence explicitement. Outre l'hexafluoropropène, les alcènes halogénés indiqués dans ce document peuvent être convertis d'une manière analogue au présent procédé, procédé au cours duquel ils réagissent avec un rendement environ aussi élevé que celui de l'hexafluoropropène.

Les exemples de formes de réalisation ci-après expliquent le procédé selon l'invention.

Exemple 1

Conformément à la figure 1, on monte un appareil en verre de borosilicate, équipé de colonnes à bulles, dans lequel le tube de réaction *T* est constitué par deux parties munies d'une enveloppe et qui peuvent être chauffées, possédant respectivement une longueur de 120 cm et un diamètre interne de 5 cm, et les résistances hydrauliques *C1, C2* et *C3* sont des plaques de polypropylène ayant une épaisseur de 0,5 cm et présentant respectivement quatre alésages axiaux ayant un diamètre de 0,3 cm. Le tube de recyclage *R* et le conduit d'alimentation *E* en HF sont constitués par de l'acier inoxydable. On remplit cet appareil jusqu'à une hauteur d'environ 25 cm au-dessus de la résistance hydraulique *C3* avec le catalyseur liquide $[(n\text{-}C_4H_9)_3N \bullet 2{,}2\ HF]$, que l'on réchauffe ensuite à 75°C via de l'eau chaude et que l'on fait circuler en circuit fermé avec un débit de 380 l/h à l'aide de la pompe de circulation *P1*. La vitesse d'écoulement du catalyseur dans le tube de réaction *T* s'élève par conséquent à 5,4 cm/s et la vitesse d'écoulement à l'intérieur des alésages des résistances hydrauliques s'élève à 368 cm/s.

A partir d'une bouteille de réserve sous pression, on introduit ensuite de l'hexafluoropropène avec un débit de 500 g/h (correspondant à 25 g/($cm^2$.h)) sous forme gazeuse au bas de la colonne. Les grosses bulles de gaz qui se forment à l'endroit du dispositif d'introduction sont finement divisées lors de leur passage à travers la résistance hydraulique *C1* et sont soumises à une décomposition pratiquement complète lors de la poursuite de leur déplacement jusqu'à la résistance hydraulique *C2.* A la résistance hydraulique *C2*, on obtient à nouveau une grande quantité de bulles de gaz finement divisées qui néanmoins, après environ 50 cm de leur déplacement ultérieur jusqu'à la résistance hydraulique *C3*, subissent une décomposition complète. Les bulles de gaz à nouveau finement divisées, qui se forment à la résistance hydraulique *C3* aboutissent rapidement à la surface de liquide dans la partie de tête *H* de la colonne à bulles. A cet endroit, le gaz est libéré sous forme d'un courant régulier, il est guidé via un conduit tubulaire dans un piège refroidi à la neige carbonique et il s'y condense. Le rendement est quantitatif, étant donné que, même après une mise en service de plusieurs heures, on n'observe aucune augmentation du volume du catalyseur liquide et on n'est confronté à aucun dégagement de gaz dans la pompe de circulation. Via le conduit d'alimentation *E*, le fluorure d'hydrogène consommé au cours de la conversion est remplacé en continu à l'aide de la pompe à membrane *P2* à partir d'un récipient de réserve en acier.

Le produit rassemblé dans le piège de refroidissement est soumis à une analyse par chromatographie gazeuse et identifié comme étant du 2-H-heptafluoropropane pratiquement pur. Par la suite, on trouve des composants élués à point d'ébullition plus élevé en une concentration totale de 314 ppm. On ne détecte pas d'hexafluoropropène.

Exemple 2

Dans cette expérience par ailleurs réalisée comme décrit à l'exemple 1, on introduit l'hexafluoropropène à un débit pratiquement deux fois plus rapide de 970 g/h (correspondant à 49 g/($cm^2$.h)). Dans le tube de réaction, entre les résistances hydrauliques *C2* et *C3*, contrairement à l'expérience de l'exemple 1, on n'observe pas de dissolution complète du gaz, mais une distribution uniforme de bulles plus grosses. Le rendement est quantitatif et on obtient des composants à points d'ébullition plus élevés, à concurrence de 457 ppm. On ne détecte pas d'hexafluoropropène.

Exemple 3

Dans une expérience par ailleurs réalisée comme décrit à l'exemple 1, on introduit l'hexafluoropropène à un débit de 1800 g/h (correspondant à 92 g/($cm^2$.h)). Le tube de réaction total *T* reste rempli de manière dense avec des

bulles de gaz finement divisées tout au long de cette expérience. Le rendement est à nouveau quantitatif; on trouve des composants à points d'ébullition plus élevés, en une concentration de 361 ppm, ainsi que de l'hexafluoropropène n'ayant pas réagi à concurrence de 53 ppm.

Exemple 4

Dans une expérience, par ailleurs réalisée comme décrit à l'exemple 2, la température du catalyseur liquide en circulation s'élève seulement à 66°C. Néanmoins, le résultat est pratiquement le même: le gaz de produit est également libéré sous forme quantitative; on trouve des composants à points d'ébullition plus élevés, à concurrence de 334 ppm, et on ne détecte pas d'hexafluoropropène dans le chromatogramme gazeux.

Exemple de comparaison

Dans un appareil équipé de colonnes à bulles, tel que décrit à l'exemple 1, toutefois, sans les résistances hydrauliques désignées par *C1-C3* en figure 1, on encastre dans la partie de base *B* pour la dispersion du gaz, un agitateur à pales à vitesse de rotation réglable. La température dans le milieu réactionnel se situe toujours à 75-79°C. Dans le tableau 1, on représente la concentration déterminée par chromatographie en phase gazeuse de l'hexafluoropropène n'ayant pas réagi dans le gaz de produit en fonction de trois paramètres réactionnels réglables différents.

Tableau 1

| Débit de recyclage (l/h) | Débit de l'hexafluoropropène (g/h) | Vitesse de rotation de l'agitateur ($min^{-1}$) | Hexafluoropropène dans le gaz de produit (ppm) | Remarques |
|---|---|---|---|---|
| 500 | 200 | 4000 | - | a) |
| 500 | 200 | 3000 | - | |
| 500 | 200 | 2000 | 133 | |
| 500 | 200 | 1500 | 919 | b) |
| 500 | 400 | 3000 | 92 | |
| 1000 | 400 | 3000 | 314 | c) |
| 500 | 840 | 3000 | 2182 | d) |
| 500 | 880 | 4000 | 461 | |
| 500 | 840 | 5000 | 125 | |
| 1000 | 200 | 3000 | 0 | |
| 500 | 400 | 4000 | 12 | |

Les remarques concernant les mises en oeuvre individuelles des essais de comparaison sont les suivantes :

a) On ne peut effectuer aucune mesure des concentrations d'hexafluoropropène dans le gaz de produit, étant donné que le mélange de réaction est sursaturé en hexafluoropropène.
b) A cause de la vitesse de rotation minime, la répartition du gaz dans la solution réactionnelle est trop mauvaise pour obtenir une conversion complète.
c) A cause du débit de recyclage élevé, le temps de séjour est trop court en l'occurrence pour obtenir une conversion complète.
d) Dans ce cas-ci, le débit pour une conversion complète est trop élevé. En général, on travaille à des vitesses de rotation inférieures à 4000 $min^{-1}$, étant donné que des vitesses de rotation supérieures consomment beaucoup d'énergie.

## Revendications

1. Procédé pour la préparation du 2-H-heptafluoropropane à partir d'hexafluoropropène et de fluorure d'hydrogène dans un appareil équipé de colonnes à bulles remplies avec, à titre de milieu réactionnel, un hydrofluorure liquide

d'une base azotée organique répondant à la formule générale

$$[B \bullet nHF] \quad (II)$$

où B représente une base azotée organique et n représente un nombre entier ou une fraction ≤ 4, caractérisé en ce qu'on utilise une colonne à bulles munie d'un système de recyclage du milieu réactionnel et comprenant une partie de base (P), un tube de réaction (*T*) et une partie de tête (*H*), dans lequel le tube de réaction (T) est équipé d'au moins une résistance hydraulique provoquant une accélération de l'écoulement du milieu réactionnel liquide d'un facteur d'au moins 20 lors de son passage à travers celle-ci.

**2.** Procédé selon la revendication 1, dans lequel la résistance hydraulique entraîne une accélération de l'écoulement du milieu liquide d'un facteur de 50 à 100.

**3.** Procédé selon la revendication 1 ou 2, dans lequel on utilise comme résistante hydraulique une plaque disposée transversalement dans le tube de réaction (T) et percée d'alésages disposés selon l'axe du tube de réaction (T).

**4.** Procédé selon la revendication 3, dans laquelle les alésages sont constitués d'orifices cylindriques, présentant une longueur au moins égale et au maximum double de leur diamètre.

**5.** Procédé selon l'une quelconque des revendications 1 à 4, dans lequel le tube de réaction (T) est équipé d'au moins deux résistances hydrauliques.

**6.** Procédé selon une quelconque des revendications 1 à 5, dans lequel on fait circuler le milieu réactionnel dans le tube de réaction (T) avec une vitesse d'écoulement de 1 à 20 cm/s.

**7.** Procédé selon une quelconque des revendications 1 à 6, dans lequel la vitesse d'écoulement du milieu réactionnel lors de son passage à travers une résistance hydraulique s'élève au moins à 200 cm/s.

**8.** Procédé selon une quelconque des revendications 1 à 7, dans lequel la hauteur du niveau de liquide dans la colonne à bulles se situe entre 2,5 et 4 m.

**9.** Procédé selon une quelconque des revendications 1 à 8, dans lequel on maintient une surpression dans la partie de base (P) du réacteur inférieure à 0,7 bar.

**Patentansprüche**

**1.** Verfahren für die Herstellung von 2-H-Heptafluorpropan aus Hexafluorpropen und Fluorwasserstoff in einer Apparatur, die mit Blasensäulen ausgestattet ist, die als Reaktionsmedium mit einem flüssigen Hydrofluorid einer organischen Stickstoffbase gefüllt sind, das der allgemeinen Formel

$$[B \cdot nHF] \quad (II)$$

entspricht, worin B eine organische Stickstoffbase darstellt und n eine ganze Zahl oder einen Bruch ≤ 4 darstellt, dadurch gekennzeichnet, dass man eine Blasensäule verwendet, die mit einem System zur Rückführung des Reaktionsmediums ausgerüstet ist und die ein Basisteil (P), ein Reaktionsrohr (*T*) und ein Kopfstück (*H*) umfasst, bei dem das Reaktionsrohr (T) mit wenigstens einem Strömungswiderstand ausgestattet ist, der eine Beschleunigung der Strömung des flüssigen Reaktionsmediums um einen Faktor von wenigstens 20 bei seinem Durchfluss durch diesen bewirkt.

**2.** Verfahren gemäß Anspruch 1, bei dem der Strömungswiderstand zu einer Beschleunigung der Strömung des flüssigen Mediums um einen Faktor von 50 bis 100 führt.

**3.** Verfahren gemäß Anspruch 1 oder 2, bei dem man als Strömungswiderstand eine Platte verwendet, die quer in dem Reaktionsrohr (T) angebracht und mit Bohrungen, die gemäß der Achse des Reaktionsrohrs (T) angeordnet sind, durchbohrt ist.

**4.** Verfahren gemäß Anspruch 3, bei dem die Bohrungen aus zylindrischen Öffnungen bestehen, die eine Länge von wenigstens gleich und höchstens dem Doppelten ihrem/s Durchmesser/s aufweisen.

**5.** Verfahren gemäß einem der Ansprüche 1 bis 4, bei dem das Reaktionsrohr (T) mit wenigstens zwei Strömungswiderständen ausgestattet ist.

**6.** Verfahren gemäß einem der Ansprüche 1 bis 5, bei dem man das Reaktionsmedium in dem Reaktionsrohr (T) mit einer Strömungsgeschwindigkeit von 1 bis 20 cm/s strömen lässt.

**7.** Verfahren gemäß einem der Ansprüche 1 bis 6, bei dem die Strömungsgeschwindigkeit des Reaktionsmediums bei seinem Durchfluss durch einen Strömungswiderstand wenigstens 200 cm/s beträgt.

**8.** Verfahren gemäß einem der Ansprüche 1 bis 7, bei dem die Höhe des Flüssigkeitspegels in der Blasensäule zwischen 2,5 und 4 m liegt.

**9.** Verfahren gemäß einem der Ansprüche 1 bis 8, bei dem man in dem Basisteil (P) des Reaktors einen Überdruck von kleiner 0,7 bar aufrechterhält.

## Claims

**1.** Process for the preparation of 2H-heptafluoropropane from hexafluoropropene and hydrogen fluoride in an apparatus equipped with bubble columns filled with, as reaction medium, a liquid hydrofluoride of an organonitrogen base corresponding to the general formula

$$[B \cdot nHF] \qquad (II)$$

where B represents an organonitrogen base and n represents an integer or a fraction $\leq 4$, characterized in that a bubble column fitted with a system for recycling the reaction medium is used, comprising a base part (P), a reaction tube (T) and a head part (H), wherein the reaction tube (T) is equipped with at least one flow resistance giving rise to an acceleration of the flow of the liquid reaction medium by a factor of at least 20 as it passes through this resistance.

**2.** Process according to Claim 1, in which the flow resistance gives rise to an acceleration of the flow of the liquid medium by a factor of 50 to 100.

**3.** Process according to Claim 1 or 2, in which a plate placed transversely in the reaction tube (T) and pierced with bores arranged along the axis of the reaction tube (T) is used as flow resistance.

**4.** Process according to Claim 3, in which the bores consist of cylindrical holes having lengths which are at least equal and at most twice their diameter.

**5.** Process according to any one of Claims 1 to 4, in which the reaction tube (T) is equipped with at least two flow resistances.

**6.** Process according to any one of Claims 1 to 5, in which the reaction medium is circulated in the reaction tube (T) at a flow rate of from 1 to 20 cm/s.

**7.** Process according to any one of Claims 1 to 6, in which the flow rate of the reaction medium as it passes through a flow resistance is at least 200 cm/s.

**8.** Process according to any one of Claims 1 to 7, in which the height of the level of liquid in the bubble column is between 2.5 and 4 m.

**9.** Process according to any one of Claims 1 to 8, in which an excess pressure below 0.7 bar is maintained in the base part (P) of the reactor.

Fig. 1